Europäisches Patentamt

European Patent Office

Office européen des brevets

(19)

(11) **EP 1 584 334 A2**

(12) # EUROPEAN PATENT APPLICATION

(43) Date of publication:
**12.10.2005 Bulletin 2005/41**

(51) Int Cl.[7]: **A61K 31/221**, A61P 11/00,
A61P 11/06

(21) Application number: **04255015.2**

(22) Date of filing: **20.08.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **01.04.2004 CA 2463001**

(71) Applicant: **Methapharm Inc.**
**Ontario N3T 2V6 (CA)**

(72) Inventor: **Truong, Van Hung**
**Westerville Ohio 43082 (US)**

(74) Representative: **Bentham, Andrew**
**J.A. Kemp & Co.**
**14 South Square**
**Gray's Inn**
**London WC1R 5JJ (GB)**

(54) **Solution based methacholine formulations**

(57)     The present invention provides inhalable solution based formulations of methacholine that are stable, sterile and ready-to-use. Also provided are methods for preparing the ready-to-use methacholine formulations. The formulations are useful in methacholine challenge testing for assessment of airway hyper-responsiveness in a subject.

EP 1 584 334 A2

**Description**

## FIELD OF THE INVENTION

The present invention pertains to chemically and physically stable, sterile pharmaceutical compositions containing methacholine and particularly to compositions formulated as a solution in a unit dose or multi-dose vials for inhalation in bronchoprovocation testing.

## BACKGROUND

Methacholine is a synthetic derivative of the neurotransmitter acetylcholine that is commonly used in bronchoprovocation tests for the evaluation of non-specific airway responsiveness. Methacholine challenge testing (MCT) is the principal test used to measure bronchial hyper-responsiveness and, therefore, is a key laboratory test used in the diagnosis of asthma (e.g. Hargreave, F.E. et al (1981) *J Allergy Clin Immunol,* 68:347-355).

Methacholine is a potent bronchoconstrictor which causes a greater degree of airway narrowing in patients with asthma compared to non-asthmatics. The patient's pulmonary function (e.g. $FEV_1$) is measured after each dose until the drop in pulmonary function exceeds a certain degree (e.g. 20% drop in $FEV_1$), or a maximal dose of methacholine has been given. The results of MCT are used by physicians to assess the airway hyper-responsiveness of the subject, which may then be used to diagnose disorders such as asthma. In the diagnosis of asthma, MCT is also useful in providing a measure of the severity of the disease. A specific application of MCT is in the confirmation or diagnosis of occupational asthma.

The widespread use of MCT is limited for a number of reasons as outlined herein. The test is technically difficult to perform and time consuming since it is performed by having the subject use a nebulizer or spirometer to inhale up to about 10 different ascending concentrations of methacholine (usually doubling doses), which are prepared at or near the time of performing the test. Methacholine is an extremely hygroscopic material and caution during solution preparation is required, for example, by handling the methacholine powder in a very low humidity environment. Once the methacholine is in solution, a technician must prepare solutions having different concentrations by sequential dilution, which is time consuming, prone to error and, therefore, costly. In addition, because of the need to prepare various solutions of methacholine, there is the possibility of errors in preparation of the solutions and, consequently, errors in the administration of correct doses. Also, because of the need to prepare the various solutions from a non-sterile bulk powder there is the possibility that the solutions will not be sterile. The potential for bacterial contamination is a serious concern that must be considered. Since the drug is administered by inhalation bacterial contamination of the formulations can seriously affect the patient's health. This is a particular concern when the solutions are to be stored for any length of time.

In addition to the foregoing, the possibility of errors in preparation of the solutions can lead to a patient receiving an inappropriately high dose, which can lead to severe bronchoconstriction. Similarly, there is a significant potential for injury to the technician involved in the preparation of the solutions from solid methacholine or from a concentrated bulk solution of methacholine.

Typically, solutions in the range of from 0.025 mg/ml to 25.0 mg/ml are employed in MCT. While solutions of > 0.3 mg/ml may be stable for at least five months when stored at 4°C, solutions having lower concentrations of methacholine exhibit reduced stability (Wanger, J.S. et al. (2003) American Thoracic Society International Annual Meeting - Seattle, Washington, A046 [Poster E32])). As a result of the observed instability of methacholine solutions it has been necessary to prepare solutions used in MCT at the time of testing. Alternatively, the solutions are prepared for a test and any left over solution is stored for only a short period of time.

Juniper, E. F. et al observed that methacholine in solution does not release free hydrogen ions (in Juniper, E. F. et al "Histamine and Methacholine Inhalation Tests: Tidal Breathing Method" Canadian Thoracic Society, Astra Draco AB, Lund Sweden, 1994). As a result, the authors suggested that the pH of methacholine solutions is stable at all concentrations and a buffered diluent is not required.

Methacholine will, however, rapidly decompose as a result of hydrolysis as the pH of the solution increase above 6. Furthermore, it has been found that solutions having lower concentrations of methacholine lose potency faster when stored at room temperature than solutions having higher concentrations (Watson, B.L. et al (1998) *Respir. Med.* 92:588-592; and Rosenfeld, J. et al. (1984) *J. Chromatogr.* 287:433-437). Watson et al. also demonstrated that solutions with a methacholine concentration of between 2.5 to 20 mg/ml exhibit improved stability at pH values of 4, 5 and 6 when stored at 4°C or 27°C, in comparison to similar solutions having pH values above 6. Watson et al. made no distinction between the effect of pH values within the range tested or between the buffer systems tested. Stability studies were not performed on solutions that have methacholine concentrations below 2.5 mg/ml.

Another study performed to evaluate the effect of different storage conditions on the stability of methacholine solutions found that methacholine was more stable when dissolved in saline than in phosphate buffered saline (Hayes, R.D. et al (1998) *Eur. Respir. J.* 11:946-948). The pH of the saline solutions containing methacholine at a concentration of 50 mg/ml and 0.39 mg/ml was 2.7 and 3.9, respectively. The authors recommended against the use of saline as a diluent, despite the improved stability, since it results in methacholine solutions having pH values that may be sufficiently low to cause bronchoconstriction by virtue of the pH alone.

In view of the foregoing, there remains a need for stable, sterile methacholine formulations that are at concentrations suitable for use in bronchoprovocation testing without further dilution, and which would overcome the difficulties described above.

This background information is provided for the purpose of making known information believed by the applicant to be of possible relevance to the present invention. No admission is necessarily intended, nor should be construed, that any of the preceding information constitutes prior art against the present invention.

## SUMMARY OF THE INVENTION

An object of the present invention is to provide a sterile solution based inhalation formulation of methacholine. In particular, it is an object of the present invention to provide inhalation formulations of methacholine that are stable and ready-to-use, for example, in bronchoprovocation testing as well as a process for the manufacture of such formulations.

In accordance with an aspect of the present invention, there is provided an inhalable, stable formulation comprising methacholine chloride at a concentration of from 0.025 to 25 mg/ml dissolved in a buffer solution having a pH in the range of about 4 to about 5.

In accordance with another aspect of the present invention, there is provided a method of preparing an inhalable, stable formulation comprising methacholine chloride at a concentration of from 0.025 to 25 mg/ml dissolved in a buffer solution having a pH in the range of about 4 to about 5, said method comprising the steps of: (a) dissolving methacholine chloride in the buffer solution; and (b) sterilising the solution formed in step (a) by aseptic filtration.

In accordance with another aspect of the present invention, there is provided a packaged pharmaceutical product comprising an inhalable, stable formulation comprising methacholine chloride at a concentration of from 0.025 to 25 mg/ml dissolved in a buffer solution having a pH in the range of about 4 to about 5, in a sealable sterile container.

In accordance with another aspect of the present invention, there is provided a use of an inhalable, stable formulation comprising methacholine chloride at a concentration of from 0.025 to 25 mg/ml dissolved in a buffer solution having a pH in the range of about 4 to about 5, for performing bronchoprovocation testing on a subject.

In accordance with another aspect of the present invention, there is provided a method for determining the amount of methacholine chloride present in a sample comprising the steps of: (a) injecting an aliquot of the sample into a high pressure liquid chromatography (HPLC) column; (b) monitoring the eluent from the HPLC column and estimating a peak area for methacholine from the sample; (c) injecting an aliquot of a standard solution of methacholine chloride into the HPLC column, which standard solution contains a known concentration of methacholine chloride; (d) monitoring the eluent from the HPLC column and estimating a peak area for methacholine from the standard solution; and, subsequently, (e) calculating from the peak areas obtained in steps (b) and (d) the amount of methacholine chloride in the sample, wherein, the HPLC column is a reverse phase column and the column is eluted using a mobile phase comprising tetramethylammonium chloride.

## BRIEF DESCRIPTION OF THE FIGURES

Figure 1 depicts an HPLC chromatogram of methacholine chloride.

Figure 2 graphically depicts the results of a comparison between the pharmaceutical effect of formulations according to the present invention that of conventional formulations.

## DETAILED DESCRIPTION OF THE INVENTION

The present invention provides stable, ready-to-use formulations of methacholine. The compositions of the present invention are formulated using acetate buffer comprising approximately 4.5 - 8.5 mM acetate and having a pH between 4 and 5. In addition, the formulations can be prepared without added preservative agent(s).

As used herein, the term "ready-to-use" refers to sterile formulations of methacholine in which methacholine is present at concentrations that are suitable for use in bronchoprovocation testing without mixing, dilution or any modification prior to administration to the patient.

The formulations of the present invention comprise methacholine in the form of a pharmaceutically acceptable salt such as, but not limited to, chloride or bromide. It should be understood that any reference to "methacholine" throughout this application is intended to refer to such pharmaceutically acceptable salts of methacholine.

In a specific embodiment, the stable, ready-to-use formulations of the present invention contain methacholine at a concentration that is suitable for use in MCT. In the USA, the concentration of methacholine used in MCT is typically within the range of between 0.025 mg/ml to 25 mg/ml methacholine. Further, the American Thoracic Society guidelines suggest the use of methacholine formulations having methacholine concentrations between 0.0625 mg/ml and 16 mg/ml and further include recommended dosing schedules. In Canada, solutions having concentrations within the range of 0.03 mg/ml to 16 mg/mL methacholine have been approved by the Therapeutic Products Directorate of Health Canada, for use in bronchoprovocation testing. The formulations of the present invention can be prepared in accordance with a predetermined dosing schedule, such as those recommended by the American Thoracic Society, which are as follows:

*Two-minute tidal breathing dosing:*

0.03, 0.06, 0.125, 0.25, 0.5, 1, 2, 4, 8, 16 mg/ml

*Five-breath dosimeter:*

0.0625, 0.25, 1, 4, 16 mg/ml

It should be appreciated that recommended dosing schedules may change since organisations such as the American Thoracic Society review their recommendations from time to time and may suggest amendments. In addition, while the guidelines for MCT are similar in different countries, the recommended dosing schedules may differ. Variations in dosing concentrations fall within the scope of the present invention, such that the ready-to-use methacholine formulations can be prepared according to any recommended dosing schedule in which the concentration of methacholine falls within the range of 0.025 - 25 mg/ml. Formulations having methacholine concentrations above 25 mg/ml can be provided but will likely be useful only as bulk solutions for the preparation of more dilute dosing formulations.

The formulations of the present invention comprise a buffer solution having a pH between about 4 and about 5. In one embodiment of the present invention the pH of the buffer solution is between 4.5 and 5.0 or, advantageously, it is between 4.5 and 4.7. In accordance with the present invention, a "buffer solution" is a solution of any acidic or basic agent having sufficient buffering capacity to maintain the pH of the solution within about 4 and about 5. The buffer solution is a solution that, by virtue of its pH, is capable of stabilising methacholine solutions and thereby preventing or reducing the formation of impurities by degradation of the methacholine. As would be readily appreciated by a worker skilled in the art, the acidic or basic agent used in the formulations of the present invention is one that does not independently cause bronchoirritation or bronchoconstriction. The term "buffer solution" thus includes solutions of a buffering agent, such as acetate or citrate, or salts thereof, and/or mixtures thereof. The buffer solution is preferably an acetate buffer solution.

In general, the buffer solution is prepared by dissolving a suitable amount of at least one buffering agent, or a salt thereof, in a pharmaceutically acceptable diluent. Typically the diluent will be water, which is preferably sterile water. The amount of buffering agent dissolved in the diluent is dependent on the type of agent used and the pharmaceutically acceptable concentrations of that agent. The amount of the buffering agent should be as high as possible, in order to maximise buffering capacity, without going beyond pharmaceutically acceptable limits. Such pharmaceutically acceptable limits will change depending on the nature of the buffering agent. Following dissolution of the buffering agent in the diluent, the pH is verified to be within the range of about 4 and about 5. If the pH is not within this range then the pH is adjusted to a pH value between about 4 and about 5 using standard techniques.

In accordance with a specific embodiment of the present invention, the buffer solution is an acetate buffer solution having an acetate concentration within the range of about 4.5 - 8.5 mM, which is advantageously about 4.8 mM. The acetate buffer can be prepared using any pharmaceutically acceptable acetate salt, such as, but not limited to, anhydrous sodium acetate, sodium acetate trihydrate and potassium acetate.

In accordance with another aspect of the present invention there is provided a method for the preparation of a stable, ready-to-use methacholine formulation. The method comprises the step of preparing a buffer solution having a pH of between about 4 and about 5. The buffered solution may be prepared using standard methods known to workers skilled in the art. For example, a 4.8 mM acetate buffer solution can be prepared from sodium acetate trihydrate by first dissolving an amount of sodium acetate trihydrate in an appropriate volume of water to produce a solution containing approximately 0.07% by weight sodium acetate trihydrate and then adjusting the pH of the solution, using an acetic acid or sodium hydroxide solution, to a pH value between 4 and 5. Typically, a 1N acetic acid or 1N sodium hydroxide solution is used for pH adjustments. Advantageously, the pH is adjusted to a pH value of between 4.5 and 5.0 or, more advantageously, between 4.5 and 4.7.

Once the buffered solution is prepared, sodium chloride may be added, if necessary, for example, to ensure that the solution has an osmolality suitable for use of the final formulation in bronchoprovocation testing. In accordance with one embodiment of the present invention, the target osmolality for a lower concentration (i.e. 0.0625 mg/ml) is 280 and the target osmolality for a higher concentration (i.e. 16 mg/ml) is 480. It has also now been found that NaCl has the added effect of assisting in the stabilization of methacholine solutions at low pH, wherein "low pH" is considered to be pH 5 and lower. The presence of the NaCl helps to maintain the pH of the formulation within the pH range of from 4 to 5.

Methacholine in the form of a pharmaceutically acceptable salt such as, but not limited to, chloride or bromide, is then added to the buffered solution. In a specific embodiment of the present invention the pharmaceutically acceptable salt is methacholine chloride. Methacholine chloride should be stored with a desiccant and weighed under dry and inert conditions (e.g nitrogen) with a very low relative humidity since it is extremely hygroscopic. Once the methacholine salt has been completely dissolved in the solution, the resulting formulation is filter sterilised and dispensed into appropriate containers with a suitable closure to maintain sterility.

In one embodiment of the present invention a bulk formulation is prepared and subsequently diluted using the acetate buffered solution (containing sodium chloride, if used) to the desired concentration. The dilution step may be performed before or after aseptic filter sterilisation. In the case where filter sterilisation is performed prior to dilution, the acetate buffered solution used for dilution is first filter sterilised and the dilution is performed using aseptic techniques.

Since the formulations of the present invention are filter sterilised and maintained under aseptic conditions during any subsequent dilution step, addition of a preservative agent is not necessary. When the formulations are packaged for multiple dosing, rather than a single dose use, it may be desirable to add a preservative agent to the final concentrations.

Preferably, the formulations of the present invention are formulated as solutions for inhalation and packaged in sealed unit dose or multi-dose containers, such as vials or ampoules, which are optionally labelled, and/or stored, with directions for use of the formulations in bronchoprovocation testing. The containers used for storage of the methacholine formulations of the present invention must consist of a material that is non-reactive (i.e. does not react with methacholine) and resistant to damage or deterioration due to long term exposure to a solution having a pH between 4 and 5. For example, the formulations of the present invention can be stored in an amber glass vial or a clear glass vial, such as a Type I, glass vial, or a low density polyethylene (LDPE) unit dose nebule, such as those used for ipratropium or salbutamol inhalation solutions.

A preferred vial volume is one suitable for multi-dose use, which would allow repeated withdrawal of sample. The increased stability attained with the formulations of the invention allow multi-dose solution-based formulation.

When a light sensitive component, such as benzyl alcohol is present, the vial is protected from intense light. Generally it is sufficient to store the vial in a darkened refrigerator or within an opaque box. However, the vial walls can comprise light transmission reducing materials. For example, translucent amber or brown vials or an opaque vial can be used. The selection of a suitable closure consisting of a suitable stopper and a suitable fastening device will be based on compatibility of different types of stoppers with the selected formulation.

In another embodiment of the invention there is provided a kit containing one or more unit dose or multi-dose containers each containing a formulation of the present invention having the same or different concentration of methacholine.

Once prepared, the formulations of the present invention can be tested to ensure that they meet the requirements for use, for example, in MCT. Chemical and physical characteristics such as pH, osmolality, colour and clarity can be determined using standard techniques that would be well known to technicians skilled in the field of pharmaceutical formulation. In addition, the formulations can be tested for the presence of microbial or particulate contaminants using standard techniques. Various assays can also be employed for confirming the presence and quantity of methacholine and/or chemical impurities (known or unknown) in the formulations. Such assays may be based on, for example, gas chromatographic (GC) or high pressure liquid chromatographic (HPLC) techniques. In the case of HPLC techniques, it has now been found that incorporation of tetramethylammonium chloride in the mobile phase stabilized the methacholine during assay, thereby making methacholine detection possible.

The formulations of the present invention are useful for performing bronchoprovocation testing in a subject. During bronchoprovocation testing the formulation of the present invention is aerosolised and inhaled by the subject to be tested. Such testing may be performed using, for example, a nebulizer or spirometer.

To gain a better understanding of the invention described herein, the following examples are set forth. It should be understood that these examples are for illustrative purposes only. Therefore, they should not limit the scope of this invention in any way.

## EXAMPLES

### EXAMPLE 1: Buffer System Selection

Two buffer systems were compared with an acetate buffer system to demonstrate the effectiveness of the acetate system having a pH 4. Each system was used to formulate laboratory scale quantities of a methacholine solution at a minimum (0.625 mg/ml) and a maximum (16 mg/ml) concentration. The pH levels of each buffer system were as follows:

acetate buffer (0.07%, by weight, sodium acetate trihydrate) to a formulated pH 4

citrate buffer (0.8%, by weight, sodium citrate dihydrate) to a formulated pH 5

phosphate buffer (0.85%, by weight, sodium phosphate dibasic) to a formulated pH 6

phosphate buffer (0.85%, by weight, sodium phosphate dibasic) to a formulated pH 7

phosphate buffer (1.28 %, by weight, sodium phosphate dibasic) to a formulated pH 8

Each of the formulations was stored under probable commercial (i.e. 5°C, 25°C) and elevated (i.e. 30°C, 40°C) temperature conditions and subsequently evaluated for changes in product potency and pH over weekly test intervals. The results are summarised in the following tables.

Table 1:

| Week 1 pH Data (Methacholine concentration = 0.0625 mg/ml) | | | | |
|---|---|---|---|---|
| Formulation Buffer System / pH | Storage Condition | | | |
| | 40 ± 2°C | 30 ± 2°C | 25 ± 2°C | 5 ± 3°C |
| Acetate buffer pH 4 | 4.04 | 4.05 | 4.06 | 4.05 |
| Citrate buffer pH 5 | 5.03 | 5.03 | 5.03 | 5.04 |
| Phosphate buffer pH 6 | 6.05 | 6.04 | 6.04 | 6.06 |
| Phosphate buffer pH 7 | 7.02 | 7.02 | 7.03 | 7.02 |
| Phosphate buffer pH 8 | 7.91 | 7.93 | 7.94 | 7.94 |

Table 2:

| Week 2 pH Data (Methacholine concentration = 0.0625 mg/ml) | | | | |
|---|---|---|---|---|
| Formulation Buffer System / pH | Storage Condition | | | |
| | 40 ± 2°C | 30 ± 2°C | 25 ± 2°C | 5 ± 3°C |
| Acetate buffer pH 4 | 4.14 | 4.20 | 4.18 | 4.13 |
| Citrate buffer pH 5 | 5.10 | 5.08 | 5.08 | 5.06 |
| Phosphate buffer pH 6 | 6.07 | 6.07 | 6.06 | 6.05 |
| Phosphate buffer pH 7 | 7.04 | 7.04 | 7.02 | 7.06 |
| Phosphate buffer pH 8 | 7.90 | 7.94 | 7.98 | 7.95 |

Table 3:

| Week 1 pH Data (Methacholine concentration = 16 mg/ml) | | | | |
|---|---|---|---|---|
| Formulation Buffer System / pH | Storage Condition | | | |
| | 40 ± 2°C | 30 ± 2°C | 25 ± 2°C | 5 ± 3°C |
| Acetate buffer pH 4 | 4.04 | 4.03 | 4.04 | 4.04 |
| Citrate buffer pH 5 | 5.01 | 5.02 | 5.03 | 5.03 |
| Phosphate buffer pH 6 | 5.93 | 6.01 | 6.04 | 6.06 |
| Phosphate buffer pH 7 | 6.65 | 6.87 | 6.93 | 7.02 |
| Phosphate buffer pH 8 | 6.88 | 7.20 | 7.34 | 7.71 |

Table 4:

| Week 2 pH Data (Methacholine concentration =16 mg/ml) | | | | |
|---|---|---|---|---|
| Formulation Buffer System / pH | Storage Condition | | | |
| | 40 ± 2°C | 30 ± 2°C | 25 ± 2°C | 5 ± 3°C |
| Acetate buffer pH 4 | 4.06 | 4.07 | 4.14 | 4.15 |
| Citrate buffer pH 5 | 5.01 | 5.03 | 5.03 | 5.04 |
| Phosphate buffer pH 6 | 5.83 | 5.97 | 5.96 | 6.00 |
| Phosphate buffer pH 7 | 6.45 | 6.74 | 6.83 | 6.99 |
| Phosphate buffer pH 8 | 6.66 | 7.01 | 7.15 | 7.64 |

Table 5:

| Week 1 Recovery Data (Methacholine concentration = 0.0625 mg/ml) | | | | |
|---|---|---|---|---|
| Formulation Buffer System / pH | Storage Condition | | | |
| | 40 ± 2°C | 30 ± 2°C | 25 ± 2°C | 5 ± 3°C |
| Acetate buffer pH 4 | 94.7% | 98.8% | 101.2% | 101.5% |
| Citrate buffer pH 5 | 98.0% | 91.8% | 98.2% | 93.3% |
| Phosphate buffer pH 6 | 94.7% | 96.3% | 95.3% | 92.2% |
| Phosphate buffer pH 7 | 66.4% | 83.2% | 90.4% | 95.1% |
| Phosphate buffer pH 8 | 16.8% | 52.4% | 63.4% | 88.9% |

Table 6:

| Week 2 Recovery Data (Methacholine concentration = 0.0625 mg/ml) | | | | |
|---|---|---|---|---|
| Formulation Buffer System / pH | Storage Condition | | | |
| | 40 ± 2°C | 30 ± 2°C | 25 ± 2°C | 5 ± 3°C |
| Acetate buffer pH 4 | 106.3% | 104.3% | 103.4% | 105.4% |
| Citrate buffer pH 5 | 103.7% | 103.7% | 103.8% | 103.9% |
| Phosphate buffer pH 6 | 93.5% | 100.4% | 101.3% | 104.6% |

Table 6:   (continued)

| Week 2 Recovery Data (Methacholine concentration = 0.0625 mg/ml) | | | | |
|---|---|---|---|---|
| Formulation Buffer System / pH | Storage Condition | | | |
| | 40 ± 2°C | 30 ± 2°C | 25 ± 2°C | 5 ± 3°C |
| Phosphate buffer pH 7 | 53.9% | 83.5% | 90.4% | 100.8% |
| Phosphate buffer pH 8 | 0% | 35.4% | 54.6% | 92.0% |

Table 7:

| Week 1 Recovery Data (Methacholine concentration =16 mg/ml) | | | | |
|---|---|---|---|---|
| Formulation Buffer System / pH | Storage Condition | | | |
| | 40 ± 2°C | 30 ± 2°C | 25 ± 2°C | 5 ± 3°C |
| Acetate buffer pH 4 | 102.0% | 102.0% | 102.7% | 103.9% |
| Citrate buffer pH 5 | 101.1% | 98.0% | 100.0% | 102.2% |
| Phosphate buffer pH 6 | 97.6% | 100.1% | 102.7% | 102.7% |
| Phosphate buffer pH 7 | 81.8% | 91.9% | 94.5% | 98.5% |
| Phosphate buffer pH 8 | 66.7% | 80.5% | 88.2% | 96.4% |

Table 8:

| Week 2 Recovery Data (Methacholine concentration =16 mg/ml) | | | | |
|---|---|---|---|---|
| Formulation Buffer System / pH | Storage Condition | | | |
| | 40 ± 2°C | 30 ± 2°C | 25 ± 2°C | 5 ± 3°C |
| Acetate buffer pH 4 | 102.5% | 102.6% | 103.1% | 103.1% |
| Citrate buffer pH 5 | 102.4% | 103.6% | 103.3% | 103.6% |
| Phosphate buffer pH 6 | 93.7% | 100.0% | 101.3% | 102.9% |
| Phosphate buffer pH 7 | 72.4% | 88.2% | 94.1% | 100.9% |
| Phosphate buffer pH 8 | 55.6% | 75.9% | 83.0% | 97.5% |

The data presented above demonstrates that product potency remained more robust at the lower formulated pH levels. In particular, when formulated within a pH range of 4 to 5 the potency levels (as demonstrated in the recovery data) remained essentially unchanged over time.

The pH of the 0.0625 mg/ml formulations remained essentially unchanged at all storage conditions, while the pH of the 16 mg/ml formulations remained essentially unchanged only at the lower pH values (i.e. below pH 6). Surprisingly, it has been found that the influence of the formulation pH appears to be magnified and more readily apparent in cases where the methacholine concentration is higher.

Although fluctuations in product potency were more readily apparent at the lower methacholine concentration (0.0625 mg/ml), this was likely due to limited chromatographic definition and symmetry at lower concentrations, rather than a true reduction in performance.

In summary, the data provided above demonstrates that the acetate pH 4 formulation and the citrate pH 5 formulation both exhibited good stability in terms of potency and pH characteristics.

## EXAMPLE 2: Buffer/pH Optimisation

An acetate buffer system was compared to a citrate buffer system to demonstrate the effectiveness of the acetate system.

From a review of the data presented in Example 1, the performance of the acetate buffered formulation (0.07% sodium acetate trihydrate) was similar to that of a citrate buffered formulation (0.8%). The United States' Food and Drug Administration (FDA) has an Inactive Ingredient Guide (IIG) that provides a listing of excipients, alone with minimum and maximum concentration ranges at which they are found in approved pharmaceutical drug products. The guidance, which has not been updated since 1996, continues to be reference by Agency reviewers. It is known that, prior to 1997, acetate buffers were not found in products delivered by inhalation. It was, however, common in injectable products to include sodium acetate trihydrate at levels between 0.00006 - 0.2%. The 0.07% level used in the formulations of Example 1 is well within this range. It was also known that citrate buffers found in inhalation products were present at levels that did not exceed 0.1%.

In light of the foregoing, the following formulations were prepared for comparison purposes:

methacholine formulated to a drug substance concentration of 16 mg/ml in a 4.8 mM acetate buffer (pH 4)

methacholine formulated to a drug substance concentration of 0.0625 mg/ml in a 4.8 mM acetate buffer (pH 4)

methacholine formulated to a drug substance concentration of 16 mg/ml in a 3.87 mM citrate buffer (pH 5)

methacholine formulated to a drug substance concentration of 0.0625 mg/ml in a 3.87 mM citrate buffer (pH 5)

Each of the formulated drug product configurations was stored under elevated (i.e. 40°C, 60°C) temperature conditions and subsequently evaluated for changes in product potency, impurity/degradation levels and pH following a two week storage period. The results are summarised in the following tables, wherein the range indicated for the "Known impurities" relates the amount of each individual known impurity detected by HPLC. The control sample, representing the same test formulation, was maintained at ambient (i.e. room temperature) conditions.

Table 9:

| Week 2 Storage Data (Methacholine concentration = 16 mg/ml) | | | | |
|---|---|---|---|---|
| Formulation pH / Sample Storage Condition | pH | Recovery | Acetic acid | Known Impurities |
| Acetate buffer pH 4 / Control | 4.04 | 100.4% | ND | ND |
| Acetate buffer pH 4 / 40°C | 4.01 | 99.8% | 0.39% | ND-1.0% |
| Acetate buffer pH 4 / 60°C | 3.93 | 95.7% | 1.57% | ND - 4.0% |
| Citrate buffer pH 5 / Control | 4.94 | 100.9% | ND | ND |
| Citrate buffer pH 5 / 40°C | 4.85 | 100.3% | 0.28% | ND-0.7% |
| Citrate buffer pH 5 / 60°C | 4.59 | 97.4% | 1.06% | ND - 2.7% |
| ND = Not detected | | | | |

Table 10:

| Week 2 Storage Data (Methacholine concentration = 0.0625 mg/ml) | | | |
|---|---|---|---|
| Formulation pH / Sample Storage Condition | pH | Recovery | Relative potency loss |
| Acetate buffer pH 4 / Control | 4.03 | 97.2% | - |
| Acetate buffer pH 4 / 40°C | 4.05 | 97.0% | 0.2% |
| Acetate buffer pH 4 / 60°C | 4.05 | 96.0% | 1.2% |
| Citrate buffer pH 5 / Control | 4.98 | 105.4 | - |
| Citrate buffer pH 5 / 40°C | 4.99 | 103.1% | 2.3% |
| Citrate buffer pH 5 / 60°C | 4.97 | 99.7 | 5.7% |

It should be recognised that, as a result of laboratory scale process constraints, a variation in starting potency was observed for the samples formulated at the 0.0625 mg/ml concentration. Therefore, relative potency loss was calculated in order to facilitate comparison and better assess the impact of storage conditions on product integrity.

The data provided above demonstrates that the acetate buffer system (pH 4) has a greater buffering capacity than the citrate buffer system (pH 5) when used in methacholine formulations. This was particularly apparent when considering the pH performance of the 16 mg/ml formulations stored under elevated temperature conditions.

The product stability (i.e. potency) was improved when processed to pH 5 for the 16 mg/ml formulations whereas the product stability was improved when processed to pH 4 for the 0.0625 mg/ml formulation.

Known degradation bi-products observed in samples stored under elevated temperature conditions are typically not apparent when the samples are stored under standard storage conditions (i.e. Control).

**EXAMPLE 3: Sterilisation**

The influence of terminal steam sterilisation on the chemical properties of the methacholine drug product was determined, specifically in terms of the influence of heat on the product potency and impurity levels within the formulations.

Sealed vials containing a 16 mg/ml methacholine formulation in 4.8 mM acetate buffer (pH 4) or 3.87 mM citrate buffer (pH 5) were subjected to simulated terminal steam sterilisation conditions of 121°C for 30 minutes. A formulation having a relatively high concentration of methacholine was used so that any changes in chemical properties were readily apparent. As would be appreciated by a worker skilled in the art, the behaviour of the formulations would be comparable throughout the methacholine concentration range. The results for both steam sterilised at 121 °C for 30 minutes and unsterilised units ("control") are summarised in the following table:

Table 11:

| Terminal Sterilisation Data | | | | |
|---|---|---|---|---|
| Sample Description | pH | Recovery | Acetic acid | Known impurities |
| Acetate buffer pH 4 / Control | 4.04 | 100.4% | ND | ND |
| Acetate buffer pH 4 / steam sterilised | 4.01 | 99.2% | 0.31% | ND - 0.8% |
| Citrate buffer pH 5 / Control | 4.94 | 100.9% | ND | ND |
| Citrate buffer pH 5 / steam sterilised | 4,70 | 98.4% | 0.71% | ND - 1.8 % |
| ND = Not detected | | | | |

Measurable potency loss was observed, with chromatographically detected known impurity levels of up to 2.5% within the final product following exposure to conventional steam sterilisation conditions (121°C for 30 minutes).

In addition, two 20 L batches of methacholine chloride, at 16 mg/ml and 0.25 mg/ml without buffer, were manufactured in a production setting and terminally steam sterilized by the conventional method. The resulting product showed that the methacholine did not withstand the high temperatures in the absence of buffer solution.

In contrast, aseptic processing of the buffered formulations following filter sterilisation was found to give satisfactory formulation stability properties.

**EXAMPLE 4: Optimisation of pH**

Four formulations of methacholine in 4.8 mM acetate buffer having different pH values were compared. The formulations compared were as follows:

methacholine formulated to a drug substance concentration of 16 mg/ml in a 4.8 mM acetate buffer pH 4.5

methacholine formulated to a drug substance concentration of 16 mg/ml in a 4.8 mM acetate buffer pH 5.0

methacholine formulated to a drug substance concentration of 0.0625 mg/ml in a 4.8 mM acetate buffer pH 4.5

methacholine formulated to a drug substance concentration of 0.0625 mg/ml in a 4.8 mM acetate buffer pH 5.0

Samples of each formulation were stored at 60°C and monitored for changes in potency, pH and impurity levels over a five week period. A summary of the data collected following two and five week storage intervals is provided below in comparison with data previously obtained using formulations prepared using 4.8 mM acetate buffer pH 4.0.

Table 12:

| Formulation at pH 4.0 (methacholine concentration = 16 mg/ml) | | | | |
|---|---|---|---|---|
| **Sample Storage Conditions** | **pH** | **Recovery** | **Acetic acid** | **Known impurities** |
| Control | 4.0 | 100.4% | ND | ND |
| 60°C for two weeks | 4.0 | 95.7% | 1.6% | ND-4.0% |
| ND = Not detected | | | | |

Table 13:

| Formulation at pH 4.5 (methacholine concentration = 16 mg/ml) | | | | |
|---|---|---|---|---|
| **Sample Storage Conditions** | **pH** | **Recovery** | **Acetic acid** | **Known impurities** |
| Control | 4.5 | 103.9% | ND | ND |
| 60°C for two weeks | 4.4 | 97.7% | 0.9% | ND - 2.2% |
| 60°C for five weeks | 4.2 | 94.8% | 2.4% | ND-6.1% |
| ND = Not detected | | | | |

Table 14:

| Formulation at pH 5.0 (methacholine concentration =16 mg/ml) | | | | |
|---|---|---|---|---|
| **Sample Storage Conditions** | **pH** | **Recovery** | **Acetic acid** | **Known impurities** |
| Control | 5.0 | 102.3% | ND | ND |
| 60°C for two weeks | 4.6 | 98.2% | 1.0% | ND - 2.5% |
| 60°C for five weeks | 4.3 | 95.0% | 2.4% | ND- 6.1% |
| ND = Not detected | | | | |

Table 15:

| Formulation at pH 4.0 (methacholine concentration = 0.0625 mg/ml) | | | | |
|---|---|---|---|---|
| **Sample Storage Conditions** | **pH** | **Recovery** | **Acetic acid** | **Known impurities** |
| Control | 4.03 | 97.2% | - | - |
| 60°C for two weeks | 4.05 | 96.0% | - | - |
| ND = Not detected | | | | |

Table 16:

| Formulation at pH 4.5 (methacholine concentration = 0.0625 mg/ml) | | | | |
|---|---|---|---|---|
| **Sample Storage Conditions** | **pH** | **Recovery** | **Acetic acid** | **Known impurities** |
| Control | 4.5 | 101.6% | ND | ND |
| 60°C for two weeks | 4.5 | 98.5% | 0.8% | ND-1.9% |
| 60°C for five weeks | 4.5 | 96.1% | - | - |
| ND = Not detected | | | | |

Table 17:

| Formulation at pH 5.0 (methacholine concentration = 0.0625 mg/ml) | | | | |
|---|---|---|---|---|
| Sample Storage Conditions | pH | Recovery | Acetic acid | Known impurities |
| Control | 5.0 | 101.4% | ND | ND |
| 60°C for two weeks | 5.0 | 98.2% | 0.7% | ND-1.8% |
| 60°C for five weeks | 5.0 | 93.4% | - | - |
| ND = Not detected | | | | |

As indicated in the data presented above, there were no impurities observed in the control samples of the 16 mg/ml formulations (at any pH tested), which were stored at room temperature for two weeks. The potency profile was better at pH 4.5 and 5.0 than at pH 4.0 with comparable potency/impurity performance for the product formulated at pH 4.5 and 5.0. The pH profile was better at pH 4.0 and 4.5 than at pH 5.0, with comparable pH performance for the product formulated at pH 4.0 and 4.5.

Similar to the 16 mg/ml formulations, there were no impurities observed in the control samples of the 0.0625 mg/ml formulations (at any pH tested), which were stored at room temperature for two weeks. The potency profile was better at pH 4.0 and 4.5 that at pH 5.0. The potency data was comparable for the product formulated at pH 4.0 and 4.5. The pH profile was comparable throughout the formulated product pH range of 4.0 to 5.0. It was not necessary to determine the impurity levels since a correlation exists between potency loss and impurity levels within the formulations. The assessment of relative potency loss and pH decline among the formulations was sufficient to compare pH effect.

Overall, this study demonstrated that formulations having a pH within the range of 4.0 to 5.0 exhibited satisfactory pH and potency stability over the testing period.

**EXAMPLE 5: Long-term Stability**

The long term stability of formulations of the present invention was demonstrated by testing methacholine formulations for various properties at monthly intervals over a nine month period of time. A 24 month stability study is ongoing. A further stability study was performed over a six month period of time under accelerated conditions (i.e. 40°C storage temperature). The methacholine formulations were prepared using methacholine chloride dissolved in a 4.8 mM acetate buffer solution with a target pH of 4.5 - 4.7. The results are summarised in the following tables. These tables also identify the reference methods used to determine characteristics of the methacholine solutions. These are standard techniques well known in the field (USP 26 for standard testing methods).

The data provided below demonstrates that the formulations of the present invention exhibit good stability following long term storage, even when stored at 40°C / 75% relative humidity. Surprisingly, this data also demonstrates that the lowest concentration tested (i.e. 0.0625 mg/ml) exhibited the most stable of the formulations tested. The individual specified impurities commonly found in methacholine formulations of the prior art are not present in detectable amounts in the formulations of the present invention.

The data provided in Tables 18 - 26 was obtained using vials that were stored in an inverted position. In order to confirm that no leaching of the vials had taken place, data was also obtained using vials that had been stored in an upright position. The data obtained following storage of the formulations at 40°C, in 75% relative humidity, for 6 months in vials maintained in an upright position is provided in Table 27.

This study demonstrates that the formulations of the present invention exhibit good long term stability when stored under standard commercial conditions and under elevated conditions (i.e. 40°C with 75% relative humidity). The data provided in Table 27 further confirms that no leaching occurred when the vials were stored in the inverted position.

**Table 18: Long term stability data**

methacholine concentration = 16 mg/ml; product size – 3 ml;
test conditions – 40°C / 75% relative humidity; container – Vial, Type I Clear, 3 ml

| TEST | METHOD | 0 month | 1 month | 2 months | 3 months | 6 months |
|---|---|---|---|---|---|---|
| Appearance/ clarity | Visual | Clear liquid | Clear liquid | Clear liquid | Clear liquid | Clear liquid |
| Foreign matter | SOP CHM-240 (visual inspection) | no particulates observed | no particulates observed | no particulates observed | no particulates observed | no particulates observed |
| Particulate matter in solution: | SOP CHM-240 | | | | | |
| < 10 μm/vial | | 440 | 2490 | 483 | 131 | 2006 |
| ≥ 10 μm/vial | | 32 | 31 | 4 | 34 | 26 |
| ≥ 25 μm/vial | | 3 | 0 | 1 | 25 | 3 |
| Colour of solution | SOP CHM-240 | colourless | colourless | colourless | colourless | colourless |
| pH | SOP CHM-240 | 4.79 / 4.80 | 4.70 / 4.68 | 4.48 / 4.48 | 4.43 / 4.43 | 4.30 / 4.29 |
| Osmolality (mOsm/kg) | SOP CHM-240 | 450 | NA | NA | NA | NA |
| Largest individual unspecified impurity | SOP CHM-224 | ND | ND | ND | ND | ND |
| Individual specified impurity | SOP CHM-224 | ND – 0.23% / ND – 0.21% | ND – 1.57% / ND – 1.58% | ND – 2.88% / ND – 2.84% | ND – 3.57% / ND – 4.03% | ND – 7.85% / ND – 7.83% |
| Acetic acid | SOP CHM-224 | 0.09% / 0.08% | 0.62% / 0.62% | 1.13% / 1.13% | 1.40% / 1.59% | 3.09% / 3.08% |
| Total unspecified impurities | SOP CHM-224 | ND / ND | ND / ND | ND / ND | ND / ND | ND / ND |
| Methacholine | SOP CHM-225 | 100.8% / 100.7% | 99.0% / 98.7% | 98.0% / 97.9% | 94.5% / 94.0% | 91.7% / 91.9% |
| Net content | SOP CHM-240 | 3.2 ml/vial | 3.2 ml/vial | 3.2 ml/vial | 3.2 ml/vial | 3.2 ml/vial |
| Sterility | Current USP <71> | meets requirements | NA | NA | meets requirements | meets requirements |

ND = Not detected; NA = Not available

EP 1 584 334 A2

### Table 19: Long term stability data

methacholine concentration = 16 mg/ml; product size – 3 ml

test conditions – 25°C / 60% relative humidity; container – Vial, Type I Clear, 3 ml

| TEST | METHOD | 0 month | 1 month | 2 months | 3 months | 6 months | 9 months |
|---|---|---|---|---|---|---|---|
| Appearance/ clarity | Visual | Clear liquid | Clear liquid | Clear liquid | Clear liquid | Clear liquid | Clear liquid |
| Foreign matter | SOP CHM-240 (visual inspection) | no particulates observed | no particulates observed | no particulates observed | no particulates observed | no particulates observed | no particulates observed |
| Particulate matter in solution: | SOP CHM-240 | | | | | | |
| < 10 µm/vial | | 440 | 2741 | 809 | 160 | 1657 | 677 |
| ≥ 10 µm/vial | | 32 | 42 | 2 | 200 | 103 | 139 |
| ≥ 25 µm/vial | | 3 | 0 | 0 | 134 | 11 | 16 |
| Colour of solution | SOP CHM-240 | colourless | colourless | colourless | Colourless | Colourless | Colourless |
| pH | SOP CHM-240 | 4.79 / 4.80 | 4.76 / 4.76 | 4.67 / 4.67 | 4.66 / 4.66 | 4.63 / 4.62 | 4.55 / 4.55 |
| Osmolality (mOsm/kg) | SOP CHM-240 | 450 | NA | NA | NA | NA | NA |
| Largest individual unspecified impurity | SOP CHM-224 | ND / ND | ND / ND | ND / ND | ND / ND | ND / ND | ND / ND |
| Individual specified impurity | SOP CHM-224 | ND – 0.23% / ND – 0.21% | ND – 0.46% / ND – 0.48% | ND – 0.72% / ND – 0.67% | ND – 0.90% / ND – 0.91% | ND – 1.80% / ND – 1.77% | ND – 2.52% / ND – 2.59% |
| Acetic acid | SOP CHM-224 | 0.09% / 0.08% | 0.18% / 0.19% | 0.28% / 0.26% | 0.35% / 0.36% | 0.71% / 0.71% | 0.99% / 1.02% |
| Total unspecified impurities | SOP CHM-224 | ND / ND | ND / ND | ND / ND | ND / ND | ND / ND | ND / ND |
| Methacholine | SOP CHM-225 | 100.8% / 100.7% | 99.4% / 99.4% | 99.1% / 100.6% | 99.7% / 100.8% | 100.1% / 99.5% | 100.1% / 99.9% |
| Net content | SOP CHM-240 | 3.2 ml/vial | 3.2 ml/vial | 3.2 ml/vial | 3.2 ml/vial | 3.2 ml/vial | 3.2 ml/vial |
| Sterility | Current USP <71> | meets requirements | NA | NA | NA | NA | NA |

ND = Not detected; NA = Not available

## Table 20: Long term stability data

methacholine concentration = 16 mg/ml; product size – 3 ml

test conditions – 5°C / ambient relative humidity; container – Vial, Type I Clear, 3 ml

| TEST | METHOD | 0 month | 1 month | 2 months | 3 months | 6 months | 9 months |
|---|---|---|---|---|---|---|---|
| Appearance/ clarity | Visual | Clear liquid | Clear liquid | Clear liquid | Clear liquid | Clear liquid | Clear liquid |
| Foreign matter | SOP CHM-240 (visual inspection) | no particulates observed | no particulates observed | no particulates observed | no particulates observed | no particulates observed | no particulates observed |
| Particulate matter in solution: | SOP CHM-240 | | | | | | |
| < 10 μm/vial | | 440 | 2767 | 662 | 411 | 1604 | 1612 |
| ≥ 10 μm/vial | | 32 | 44 | 8 | 238 | 133 | 187 |
| ≥ 25 μm/vial | | 3 | 2 | 2 | 85 | 15 | 24 |
| Colour of solution | SOP CHM-240 | colourless | colourless | colourless | Colourless | Colourless | Colourless |
| pH | SOP CHM-240 | 4.79 / 4.80 | 4.83 / 4.82 | 4.71 / 4.71 | 4.73 / 4.73 | 4.80 / 4.78 | 4.75 / 4.76 |
| Osmolality (mOsm/kg) | SOP CHM-240 | 450 | NA | NA | NA | NA | NA |
| Largest individual unspecified impurity | SOP CHM-224 | ND / ND | ND / ND | ND / ND | ND / ND | ND / ND | ND / ND |
| Individual specified impurity | SOP CHM-224 | ND – 0.23% / ND – 0.21% | ND – 0.32% / ND – 0.32% | ND – 0.35% / ND – 0.35% | ND – 0.30% / ND – 0.28% | ND – 0.49% / ND – 0.47% | ND – 0.39% / ND – 0.42% |
| Acetic acid | SOP CHM-224 | 0.09% / 0.08% | 0.13% / 0.13% | 0.14% / 0.13% | 0.12% / 0.11% | 0.19% / 0.18% | 0.15% / 0.17% |
| Total unspecified impurities | SOP CHM-224 | ND / ND | ND / ND | ND / ND | ND / ND | ND / ND | ND / ND |
| Methacholine | SOP CHM-225 | 100.8% / 100.7% | 99.9% / 99.9% | 101.2% / 101.5% | 99.5% / 97.4% | 101.9% / 102.0% | 102.2% / 102.5% |
| Net content | SOP CHM-240 | 3.2 ml/vial | 3.2 ml/vial | 3.2 ml/vial | 3.2 ml/vial | 3.2 ml/vial | 3.2 ml/vial |
| Sterility | Current USP <71> | meets requirements | NA | NA | NA | NA | NA |

ND = Not detected; NA = Not available

EP 1 584 334 A2

**Table 21: Long term stability data**

methacholine concentration = 1 mg/ml; product size – 3 ml
test conditions – 40°C / 75% relative humidity; container – Vial, Type I Clear, 3 ml

| TEST | METHOD | 0 month | 1 month | 2 months | 3 months | 6 months |
|---|---|---|---|---|---|---|
| Appearance/ clarity | Visual | Clear liquid | Clear liquid | Clear liquid | Clear liquid | Clear liquid |
| Foreign matter | SOP CHM-240 (visual inspection) | no particulates observed | no particulates observed | no particulates observed | no particulates observed | no particulates observed |
| Particulate matter in solution: | SOP CHM-240 | | | | | |
| < 10 µm/vial | | 1159 | 2555 | 680 | 100 | 3887 |
| ≥ 10 µm/vial | | 6 | 28 | 4 | 156 | 124 |
| ≥ 25 µm/vial | | 0 | 1 | 3 | 93 | 11 |
| Colour of solution | SOP CHM-240 | colourless | colourless | colourless | Colourless | Colourless |
| pH | SOP CHM-240 | 4.77 4.77 | 4.76 4.76 | 4.69 4.67 | 4.70 4.70 | 4.76 4.73 |
| Osmolality (mOsm/kg) | SOP CHM-240 | 312 310 | NA | NA | NA | NA |
| Largest individual unspecified impurity | SOP CHM-224 | ND ND | ND ND | ND ND | ND ND | ND ND |
| Individual specified impurity | SOP CHM-224 | ND – 0.08% ND – 0.06% | ND – 0.55% ND – 0.53% | ND – 0.94% ND – 0.91% | ND – 1.42% ND – 1.13% | ND – 2.48% ND – 2.70% |
| Acetic acid | SOP CHM-224 | 0.03% 0.02% | 0.22% 0.21% | 0.37% 0.36% | 0.56% 0.44% | 0.98% 1.06% |
| Total unspecified impurities | SOP CHM-224 | ND ND | ND ND | ND ND | ND ND | ND ND |
| Methacholine | SOP CHM-225 | 101.3% 101.0% | 98.4% 98.8% | 98.0% 98.2% | 97.5% 95.7% | 93.1% 93.1% |
| Net content | SOP CHM-240 | 3.2 ml/vial | 3.2 ml/vial | 3.2 ml/vial | 3.2 ml/vial | 3.2 ml/vial |
| Sterility | Current USP <71> | meets requirements | NA | NA | meets requirements | meets requirements |

ND = Not detected; NA = Not available

EP 1 584 334 A2

## Table 22: Long term stability data

methacholine concentration = 1 mg/ml; product size – 3 ml

test conditions – 25°C / 60% relative humidity; container – Vial, Type I Clear, 3 ml

| TEST | METHOD | 0 month | 1 month | 2 months | 3 months | 6 months | 9 months |
|---|---|---|---|---|---|---|---|
| Appearance/ clarity | Visual | Clear liquid | Clear liquid | Clear liquid | Clear liquid | Clear liquid | Clear liquid |
| Foreign matter | SOP CHM-240 (visual inspection) | no particulates observed | no particulates observed | no particulates observed | no particulates observed | no particulates observed | no particulates observed |
| Particulate matter in solution: | SOP CHM-240 | | | | | | |
| < 10 µm/vial | | 1159 | 1836 | 461 | 277 | 3016 | 2514 |
| ≥ 10 µm/vial | | 6 | 47 | 9 | 77 | 75 | 192 |
| ≥ 25 µm/vial | | 0 | 0 | 6 | 27 | 7 | 23 |
| Colour of solution | SOP CHM-240 | colourless | colourless | colourless | Colourless | Colourless | Colourless |
| pH | SOP CHM-240 | 4.77 / 4.77 | 4.78 / 4.78 | 4.71 / 4.71 | 4.74 / 4.74 | 4.80 / 4.75 | 4.76 / 4.75 |
| Osmolality (mOsm/kg) | SOP CHM-240 | 312 / 310 | NA | NA | NA | NA | NA |
| Largest individual unspecified impurity | SOP CHM-224 | ND / ND | ND / ND | ND / ND | ND / ND | ND / ND | ND / ND |
| Individual specified impurity | SOP CHM-224 | ND – 0.08% / ND – 0.06% | ND – 0.17% / ND – 0.16% | ND – 0.26% / ND – 0.20% | ND – 0.18% / ND – 0.24% | ND – 0.74% / ND – 0.71% | ND – 1.64% / ND – 1.39% |
| Acetic acid | SOP CHM-224 | 0.03% / 0.02% | 0.07% / 0.06% | 0.10% / 0.08% | 0.07% / 0.10% | 0.29% / 0.28% | 0.65% / 0.55% |
| Total unspecified impurities | SOP CHM-224 | ND / ND | ND / ND | ND / ND | ND / ND | ND / ND | ND / ND |
| Methacholine | SOP CHM-225 | 101.3% / 101.0% | 99.2% / 99.5% | 100.4% / 100.9% | 100.9% / 100.8% | 100.2% / 99.2% | 99.5% / 99.4% |
| Net content | SOP CHM-240 | 3.2 ml/vial | 3.2 ml/vial | 3.2 ml/vial | 3.2 ml/vial | 3.2 ml/vial | 3.2 ml/vial |
| Sterility | Current USP <71> | meets requirements | NA | NA | NA | NA | NA |

ND = Not detected; NA = Not available

**Table 23: Long term stability data**

methacholine concentration = 1 mg/ml; product size – 3 ml

test conditions – 5°C / ambient relative humidity; container – Vial, Type I Clear, 3 ml

| TEST | METHOD | 0 month | 1 month | 2 months | 3 months | 6 months | 9 months |
|---|---|---|---|---|---|---|---|
| Appearance/ clarity | Visual | Clear liquid | Clear liquid | Clear liquid | Clear liquid | Clear liquid | Clear liquid |
| Foreign matter | SOP CHM-240 (visual inspection) | no particulates observed | no particulates observed | no particulates observed | no particulates observed | no particulates observed | no particulates observed |
| Particulate matter in solution: | SOP CHM-240 | | | | | | |
| < 10 µm/vial | | 1159 | 2112 | 613 | 238 | 1861 | 1064 |
| ≥ 10 µm/vial | | 6 | 36 | 11 | 193 | 125 | 198 |
| ≥ 25 µm/vial | | 0 | 0 | 4 | 110 | 11 | 27 |
| Colour of solution | SOP CHM-240 | colourless | colourless | colourless | Colourless | Colourless | Colourless |
| pH | SOP CHM-240 | 4.77 4.77 | 4.76 4.76 | 4.71 4.71 | 4.78 4.76 | 4.80 4.79 | 4.76 4.757 |
| Osmolality (mOsm/kg) | SOP CHM-240 | 312 310 | NA | NA | NA | NA | NA |
| Largest individual unspecified impurity | SOP CHM-224 | ND ND | ND ND | ND ND | ND ND | ND ND | ND ND |
| Individual specified impurity | SOP CHM-224 | ND – 0.08% ND – 0.06% | ND – 0.07% ND – 0.05% | ND ND | ND ND | ND – 0.21% ND – 0.27% | ND – 0.23% ND – 0.28% |
| Acetic acid | SOP CHM-224 | 0.03% 0.02% | 0.03% 0.02% | ND (<0.01%) ND (<0.01%) | ND (<0.01%) ND (<0.01%) | 0.08% 0.11% | 0.09% 0.11% |
| Total unspecified impurities | SOP CHM-224 | ND ND | ND ND | ND ND | ND ND | ND ND | ND ND |
| Methacholine | SOP CHM-225 | 101.3% 101.0% | 100.8% 100.6% | 101.7% 101.4% | 102.1% 101.5% | 101.4% 101.7% | 102.1% 102.1% |
| Net content | SOP CHM-240 | 3.2 ml/vial | 3.2 ml/vial | 3.2 ml/vial | 3.2 ml/vial | 3.2 ml/vial | 3.2 ml/vial |
| Sterility | Current USP <71> | meets requirements | NA | NA | NA | NA | NA |

ND = Not detected; NA = Not available

## Table 24: Long term stability data

methacholine concentration = 0.0625 mg/ml; product size – 3 ml

test conditions – 40°C / 75% relative humidity; container – Vial, Type I Clear, 3 ml

| TEST | METHOD | 0 month | 1 month | 2 months | 3 months | 6 months |
|---|---|---|---|---|---|---|
| Appearance/ clarity | Visual | Clear liquid | Clear liquid | Clear liquid | Clear liquid | Clear liquid |
| Foreign matter | SOP CHM-240 (visual inspection) | no particulates observed | no particulates observed | no particulates observed | no particulates observed | no particulates observed |
| Particulate matter in solution: | SOP CHM-240 | | | | | |
| < 10 µm/vial | | 847 | 1503 | 434 | 172 | 1385 |
| ≥ 10 µm/vial | | 10 | 28 | 9 | 168 | 106 |
| ≥ 25 µm/vial | | 1 | 0 | 5 | 88 | 12 |
| Colour of solution | SOP CHM-240 | colourless | colourless | colourless | Colourless | Colourless |
| pH | SOP CHM-240 | 4.75 / 4.75 | 4.77 / 4.75 | 4.70 / 4.70 | 4.71 / 4.71 | 4.78 / 4.76 |
| Osmolality (mOsm/kg) | SOP CHM-240 | 301 / 301 | NA | NA | NA | NA |
| Largest individual unspecified impurity | SOP CHM-224 | ND / ND | ND / ND | ND / ND | ND / ND | ND / ND |
| Individual specified impurity | SOP CHM-224 | ND / ND | ND / ND | ND – 0.72% / ND – 0.75% | ND – 0.65% / ND – 0.57% | ND – 1.04% / ND – 1.02% |
| Acetic acid | SOP CHM-224 | ND (<0.06%) / ND (<0.06%) | ND (<0.06%) / ND (<0.06%) | 0.28% / 0.30% | 0.26% / 0.22% | 0.41% / 0.40% |
| Total unspecified impurities | SOP CHM-224 | ND / ND | ND / ND | ND / ND | ND / ND | ND / ND |
| Methacholine | SOP CHM-225 | 100.9% / 101.0% | 99.9% / 99.8% | 98.1% / 98.2% | 97.9% / 96.8% | 91.8% / 91.5% |
| Net content | SOP CHM-240 | 3.2 ml/vial | 3.2 ml/vial | 3.2 ml/vial | 3.2 ml/vial | 3.2 ml/vial |
| Sterility | Current USP <71> | meets requirements | NA | NA | meets requirements | meets requirements |

ND = Not detected; NA = Not available

EP 1 584 334 A2

**Table 25: Long term stability data**

methacholine concentration = 0.0625 mg/ml; product size – 3 ml

test conditions – 25°C / 60% relative humidity; container – Vial, Type I Clear, 3 ml

| TEST | METHOD | 0 month | 1 month | 2 months | 3 months | 6 months | 9 months |
|---|---|---|---|---|---|---|---|
| Appearance/ clarity | Visual | Clear liquid | Clear liquid | Clear liquid | Clear liquid | Clear liquid | Clear liquid |
| Foreign matter | SOP CHM-240 (visual inspection) | no particulates observed | no particulates observed | no particulates observed | no particulates observed | no particulates observed | no particulates observed |
| Particulate matter in solution: | SOP CHM-240 | | | | | | |
| < 10 µm/vial | | 847 | 1299 | 340 | 170 | 1527 | 3297 |
| ≥ 10 µm/vial | | 10 | 36 | 8 | 140 | 53 | 28 |
| ≥ 25 µm/vial | | 1 | 0 | 4 | 21 | 5 | 0 |
| Colour of solution | SOP CHM-240 | Colourless | Colourless | Colourless | Colourless | Colourless | Colourless |
| pH | SOP CHM-240 | 4.75 4.75 | 4.75 4.74 | 4.71 4.70 | 4.70 4.70 | 4.79 4.76 | 4.77 4.75 |
| Osmolality (mOsm/kg) | SOP CHM-240 | 301 301 | NA | NA | NA | NA | NA |
| Largest individual unspecified impurity | SOP CHM-224 | ND ND | ND ND | ND ND | ND ND | ND ND | ND ND |
| Individual specified impurity | SOP CHM-224 | ND ND | ND ND | ND ND | ND ND | ND – 0.65% ND – 0.78% | ND – 0.89% ND – 1.05% |
| Acetic acid | SOP CHM-224 | ND (<0.06%) ND (<0.06%) | ND (<0.06%) ND (<0.06%) | ND (<0.06%) ND (<0.06%) | ND (<0.06%) ND (<0.06%) | 0.26% 0.31% | 0.35% 0.41% |
| Total unspecified impurities | SOP CHM-224 | ND ND | ND ND | ND ND | ND ND | ND ND | ND ND |
| Methacholine | SOP CHM-225 | 100.9% 101.0% | 101.8% 102.2% | 101.2% 100.4% | 102.9% 102.4% | 98.5% 98.5% | 100.0% 101.2% |
| Net content | SOP CHM-240 | 3.2 ml/vial | 3.2 ml/vial | 3.2 ml/vial | 3.2 ml/vial | 3.2 ml/vial | 3.2 ml/vial |
| Sterility | Current USP <71> | meets requirements | NA | NA | NA | NA | NA |

ND = Not detected; NA = Not available

EP 1 584 334 A2

## Table 26: Long term stability data

methacholine concentration = 0.0625 mg/ml; product size – 3 ml
test conditions – 5°C / ambient relative humidity; container – Vial, Type I Clear, 3 ml

| TEST | METHOD | 0 month | 1 month | 2 months | 3 months | 6 months | 9 months |
|---|---|---|---|---|---|---|---|
| Appearance/ clarity | Visual | Clear liquid | Clear liquid | Clear liquid | Clear liquid | Clear liquid | Clear liquid |
| Foreign matter | SOP CHM-240 (visual inspection) | no particulates observed | no particulates observed | no particulates observed | no particulates observed | no particulates observed | no particulates observed |
| Particulate matter in solution: | SOP CHM-240 | | | | | | |
| < 10 µm/vial | | 847 | 1349 | 390 | 135 | 1994 | 2534 |
| ≥ 10 µm/vial | | 10 | 67 | 14 | 262 | 25 | 122 |
| ≥ 25 µm/vial | | 1 | 0 | 4 | 110 | 2 | 20 |
| Colour of solution | SOP CHM-240 | Colourless | Colourless | Colourless | Colourless | Colourless | Colourless |
| pH | SOP CHM-240 | 4.75 / 4.75 | 4.75 / 4.75 | 4.72 / 4.73 | 4.70 / 4.70 | 4.74 / 4.74 | 4.75 / 4.75 |
| Osmolality (mOsm/kg) | SOP CHM-240 | 301 / 301 | NA | NA | NA | NA | NA |
| Largest individual unspecified impurity | SOP CHM-224 | ND / ND | ND / ND | ND / ND | ND / ND | ND / ND | ND / ND |
| Individual specified impurity | SOP CHM-224 | ND / ND | ND / ND | ND / ND | ND / ND | ND – 0.63% / ND – 0.54% | ND / ND |
| Acetic acid | SOP CHM-224 | ND (<0.06%) / ND (<0.06%) | ND (<0.06%) / ND (<0.06%) | ND (<0.06%) / ND (<0.06%) | ND (<0.06%) / ND (<0.06%) | 0.25% / 0.21% | ND (<0.06%) / ND (<0.06%) |
| Total unspecified impurities | SOP CHM-224 | ND / ND | ND / ND | ND / ND | ND / ND | ND / ND | ND / ND |
| Methacholine | SOP CHM-225 | 100.9% / 101.0% | 101.7% / 101.5% | 101.4% / 101.1% | 102.2% / 102.3% | 100.0% / 100.8% | 102.9% / 103.2% |
| Net content | SOP CHM-240 | 3.2 ml/vial | 3.2 ml/vial | 3.2 ml/vial | 3.2 ml/vial | 3.2 ml/vial | 3.2 ml/vial |
| Sterility | Current USP <71> | meets requirements | NA | NA | NA | NA | NA |

ND = Not detected; NA = Not available

EP 1 584 334 A2

**Table 27: Long term stability of formulations stored in an upright position.**

| TEST | METHOD | 16 mg/ml | 1 mg/ml | 0.625 mg/ml |
|---|---|---|---|---|
| Appearance/ clarity | Visual | Clear liquid | Clear liquid | Clear liquid |
| Foreign matter | SOP CHM-240 (visual inspection) | no particulates observed | no particulates observed | no particulates observed |
| Particulate matter in solution: | SOP CHM-240 | | | |
| < 10 µm/vial | | 1651 | 3116 | 1374 |
| ≥ 10 µm/vial | | 144 | 94 | 64 |
| ≥ 25 µm/vial | | 14 | 12 | 7 |
| Colour of solution | SOP CHM-240 | colourless | colourless | colourless |
| pH | SOP CHM-240 | 4.32 4.30 | 4.76 4.74 | 4.79 4.78 |
| Largest individual unspecified impurity | SOP CHM-224 | ND ND | ND ND | ND ND |
| Individual specified impurity | SOP CHM-224 | ND – 7.92% ND – 7.90% | ND – 2.51% ND – 2.79% | ND – 1.19% ND – 1.34% |
| Acetic acid | SOP CHM-224 | 3.12% 3.11% | 0.99% 1.10% | 0.47% 0.53% |
| Total unspecified impurities | SOP CHM-224 | ND ND | ND ND | ND ND |
| Methacholine | SOP CHM-225 | 92.0% 92.3% | 93.0% 93.0% | 91.3% 91.2% |
| Net content | SOP CHM-240 | 3.2 ml/vial | 3.2 ml/vial | 3.2 ml/vial |
| Sterility | Current USP <71> | meets requirements | meets requirements | meets requirements |

ND = Not detected; NA = Not available

EP 1 584 334 A2

**EXAMPLE 6: Methacholine Assay**

The procedure described below was used for performing the high pressure liquid chromatography (HPLC) assay analysis of methacholine chloride in solution.

*Materials and Equipment*

- analytical balance
- HPLC system
- assorted laboratory glassware
- 0.45 micron nylon membrane filter or smaller
- HPLC grade methanol
- HPLC grade water
- 1-heptansulfonic acid sodium salt
- potassium phosphate monobasic
- tetramethylammonium chloride
- methacholine chloride reference standard
- waters symmetry $C_{18}$, 3.5 $\mu$m, 4.6 $\times$ 100 mm, HPLC column (e.g. Waters # WAT066220 or equivalent)

*Procedure*

1.0 Preparation of mobile phase

1.1 Dissolve about 2.0 g of 1-heptanesulfonic acid sodium, 6.8 g of potassium phosphate monobasic and 0.53 g of tetramethylammonium chloride in 800 ml of water. Add 120 ml of methanol and mix. Cool to room temperature.

1.2 Transfer to a 1-L volumetric flask and fill to volume with water and mix well.

1.3 Degas by vacuum filtration or other suitable means and filter through a 0.45 micron or smaller nylon membrane filter.

2.0 Standard solution preparation

2.1 Methacholine chloride is extremely hygroscopic, therefore, the methacholine chloride reference standard must be weighed or pre-dispensed in a dry glove box within which a dry gas atmosphere is maintained (e.g. Argon).

2.2 Methacholine chloride standard: Transfer about 25 mg, accurately weighed or pre-dispensed, of methacholine chloride reference standard to a 50 ml volumetric flask. Dissolve and dilute to volume with mobile phase and mix well.

2.3 The resulting solution contains about 0.5 mg/ml of methacholine chloride.

2.4 Prepare in duplicate as a standard check.

3.0 Finished product sample preparation

3.1 For samples having a methacholine chloride concentration of 16 mg/ml:

3.1.1 Pipette 3 ml of finished product into a 100 ml volumetric flask. Dilute to volume with mobile phase and mix.

3.1.2 The resulting solution contains about 0.48 mg/ml of methacholine chloride.

3.1.3 Prepare in duplicate.

3.2 For samples having a methacholine chloride concentration of 4 mg/ml:

3.2.1 Pipette 3 ml of finished product into a 25-mL volumetric flask. Dilute to volume with mobile phase and mix.

3.2.2 The resulting solution contains about 0.48 mg/ml of methacholine chloride.

3.2.3 Prepare in duplicate.

3.3 For samples having a methacholine chloride concentration of 1 mg/ml:

3.3.1 Pipette 5 ml of finished product into a 10 ml volumetric flask. Dilute to volume with mobile phase and mix.

3.3.2 The resulting solution contains about 0.5 mg/ml of methacholine chloride.

3.3.3 Prepare in duplicate.

3.4 For samples having a methacholine chloride concentration of 0.25 mg/ml and 0.0625 mg/ml:

3.4.1 Inject the sample "as is"

3.4.2 Prepare in duplicate.

*Chromatographic Conditions:*

| Flow rate | about 1.1 ml/minute |
|---|---|
| Detector | Refractive index |
| Injection volume | 100 μl |
| Column temperature | 40°C |
| Run time | 12 min. |

*System Suitability:*

The expected relative retention time of methacholine chloride is about 7 minutes. The tailing factor of the methacholine peak must not exceed 3.0. The relative standard deviation of methacholine peak areas from replicate injections of standard solution may not exceed 2.0%. The second standard preparation should be within 98.0% to 102.0% of the first standard preparation.

*Test Procedure:*

Use peak area for all calculations.
Equilibrate the HPLC system with mobile phase for at least 120 minutes.
If necessary, make 1-2 injections of the sample solution to help stabilise the column.
Once the system is stabilised, make a minimum of six (6) replicate 100 μL injections of the first standard preparation solution. Two (2) injections of the second standard preparation solution should then be made.
Duplicate sample injections may be made following satisfactory evaluation of standard injection results.
Inject the first standard solution, in duplicate, after every four sample preparations (typically 8 injections) and at the end of the run.

*Calculation of Methacholine Chloride in Solution-Based Finished Product:*

1.0 For samples having a methacholine chloride concentration of 16 mg/mL:

Measure the peak responses obtained at corresponding refractive index retention times and calculate the percent label claim of methacholine in the solution-based finished product by the following formula:

$$\%L.C. = \frac{Pu}{Ps} \times \frac{Std.Wt.}{50} \times \frac{Std.pot.}{100} \times \frac{100}{3} \times \frac{100}{16}$$

Where:

Pu =  *Average* peak area of methacholine chloride in the sample
Ps =  Average peak area of methacholine chloride in the standard
Std. Wt. = Standard weight of methacholine chloride, in mg
Std. pot. = Standard potency
16 =  Label claim of methacholine chloride in the solution-based finished product, in mg/ml

2.0 For samples having a methacholine chloride concentration of 4 mg/ml:

Measure the peak responses obtained at corresponding refractive index retention times and calculate the % label claim of methacholine in the solution-based finished product by the formula:

$$\%L.C. = \frac{Pu}{Ps} \times \frac{Std.Wt.}{50} \times \frac{Std.pot.}{100} \times \frac{25}{3} \times \frac{100}{4}$$

Where:

4 = Label claim of methacholine chloride in the solution-based finished product, in mg/ml

3.0 For samples having a methacholine chloride concentration of 1 mg/ml:

Measure the peak responses obtained at corresponding refractive index retention times and calculate the % label claim of methacholine in the solution-based finished product by the formula:

$$\%L.C. = \frac{Pu}{Ps} \times \frac{Std.Wt.}{50} \times \frac{Std.pot.}{100} \times \frac{10}{5} \times \frac{100}{1}$$

Where:

1 = Label claim of methacholine chloride in the solution-based finished product, in mg/ml

4.0 For samples having a methacholine chloride concentration of 0.25 mg/ml:

Measure the peak responses obtained at corresponding refractive index retention times and calculate the % label claim of methacholine in the solution-based finished product by the formula:

$$\%L.C. = \frac{Pu}{Ps} \times \frac{Std.Wt.}{50} \times \frac{Std.pot.}{100} \times \frac{100}{0.25}$$

Where:

0.25 = Label claim of methacholine chloride in the solution-based finished product, in mg/ml

5.0 For samples having a methacholine chloride concentration of 0.0625 mg/ml:

Measure the peak responses obtained at corresponding refractive index retention times and calculate the % label claim of methacholine in the solution-based finished product by the formula:

$$\%L.C. = \frac{Pu}{Ps} \times \frac{Std.Wt.}{50} \times \frac{Std.pot.}{100} \times \frac{100}{0.0625}$$

Where:

0.0625 =     Label claim of methacholine chloride in the solution-based finished product, in mg/ml

*System Shut Down:*

Before the system is shut down, flush with 10/90 methanol/water for 120 minutes. For long-term storage, store the column in 50/50 methanol/water.

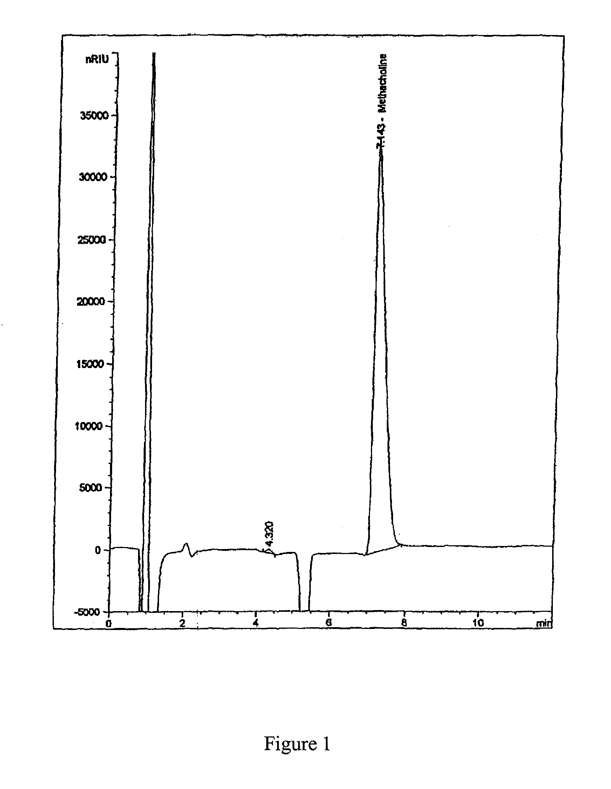

An HPLC chromatogram of the finished product is provided in Figure 1.

### EXAMPLE 7: Comparison to Conventional Methacholine Formulation

The following study demonstrates that the formulation of the present invention is pharmaceutically equivalent to a methacholine formulation prepared using a conventional isotonic saline diluent.

METHOD

*Subjects*

Twelve subjects with asthma volunteered for the study. Subjects included 5 male and 7 female, age $28.8 \pm 3.3$ yr, height $67.3 \pm 0.9$ inches. They had $FEV_1 > 70\%$ of predicted and methacholine $PC_{20}$ (tidal breathing method) < 8 mg/ml. Subjects were stable with no relevant allergen exposure, no respiratory tract infection, and no change in inhaled corticosteroid dose (n=4) for > four weeks. The study was approved by the University of Saskatchewan Ethics Committee and signed informed consent was obtained.

*Diluent Preparation*

Two diluents were used. The conventional diluent was sterile 0.9% isotonic saline. The acetate diluent was prepared using 90 mg/10 ml (0.9%) sodium chloride and 6.6 mg/10 ml sodium acetate trihydate (0.066%). Doubling concentrations of methacholine from 0.03 to 8.0 mg/ml were prepared in the two diluents, given a two-week shelf life, and stored in the fridge at 4° C.

*Methacholine Challenge*

Methacholine challenge was performed by the standard 2 minute tidal breathing method (Cockcroft, D.W., et al (1977) *Clin Allergy*, 7:235-243). Aerosols were generated from a Bennett Twin jet nebulizer calibrated to an output of 0.13 ml/minute. Subjects wore nose clips and inhaled aerosols via a loose-fitting facemask for 2 minutes of tidal breathing. Diluent was inhaled initially followed by doubling concentrations of methacholine with a 5 minute interval between the start of one concentration and the start of the next. Spirometry was initially measured in triplicate. Single measurements of $FEV_1$ from a technically acceptable truncated spirogram were measured 30 and 90 seconds after the completion of each inhalation. The percent change in $FEV_1$ was calculated from the lowest post-diluent to the lowest post-methacholine value. The test was continued until a $\geq 18\%$ fall in $FEV_1$ was obtained and the $PC_{20}$ calculated by an algebraic formula (Cockcroft, D.W., et al (1983) *Chest*, 84:505-506).

*Study Design*

Subjects attended the laboratory on two days at the same time of day within a one-week period. On each day, a methacholine challenge was done starting at the same concentration, one with saline diluent, and one with acetate diluent. The order of tests were randomized in an alternating fashion; odd numbered subjects performed the saline diluent methacholine challenge on Day 1 and even numbered subjects the acetate diluent test on Day 1. The pH of each methacholine dilution was measured for both diluents prior to inhalation.

*Analysis*

$PC_{20}$ values were log transformed for analysis. The primary endpoint was the methacholine $PC_{20}$ compared by paired t-test. We also compared by paired t-test the percent fall in $FEV_1$ following the two diluents. Based on previous data from our laboratory, this investigation had a greater than 95% power to detect a one-half concentration difference in methacholine $PC_{20}$ values; this is the minimum clinically relevant difference.

RESULTS

Demographics of the 12 subjects are given in Table 28. All 12 subjects completed the investigation with no adverse events.

Table 28:

| Subject demographics | | | | | | | |
|---|---|---|---|---|---|---|---|
| Subject | Gender | Age (years) | Height (inches) | Con meds | Baseline FEV1(L) | % predicted FEV1 | Baseline FVC (L) |
| 1 | M | 57 | 66 | S | 2.52 | 75 | 4.15 |
| 2 | F | 39 | 66 | S | 2.97 | 94 | 4.53 |
| 3 | M | 21 | 71 | S | 4.87 | 102 | 5.59 |
| 4 | F | 22 | 69 | S; F 125 bid | 3.3 | 86 | 3.61 |
| 5 | M | 24 | 70 | S; F250 bid | 3.65 | 80 | 6.17 |
| 6 | M | 25 | 72 | S | 4.03 | 85 | 5.53 |
| 7 | M | 27 | 71 | S | 3.76 | 81 | 4.81 |
| 8 | F | 23 | 66 | S;F 125 bid | 3.18 | 89 | 4.3 |
| 9 | F | 24 | 62 | S | 2.89 | 90 | 3.82 |
| 10 | F | 19 | 66 | S | 3.65 | 99 | 4.78 |
| 11 | F | 23 | 63 | T; F 125 bid | 2.71 | 82 | 3.64 |
| 12 | F | 42 | 65 | BDP; S | 3.02 | 101 | 4.05 |
| Mean | | 29 | 67 | | 3.38 | 89 | 4.58 |
| S = salbutamol | | | | | | | |
| F = fluticasone propionate | | | | | | | |
| T = terbutaline | | | | | | | |
| BDP = beclomethasone dipropionate | | | | | | | |

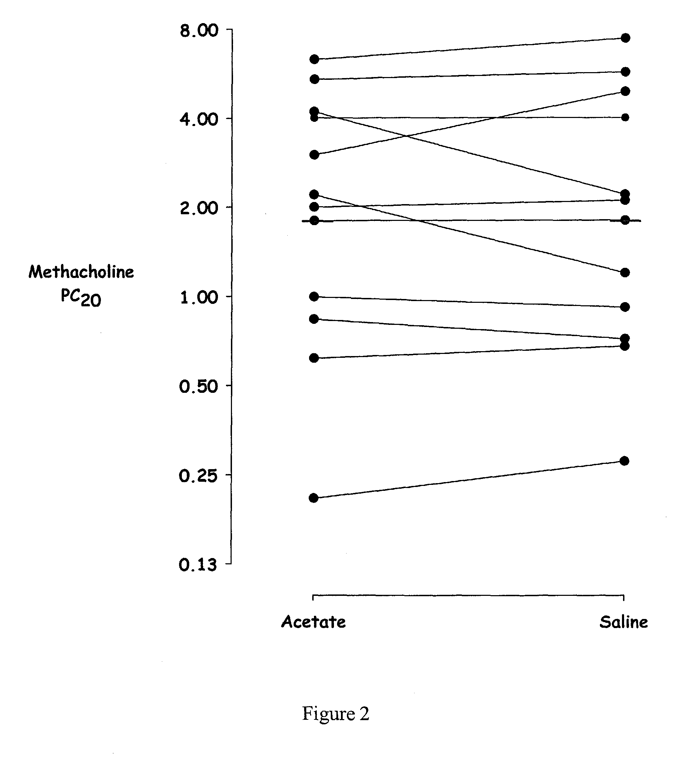

Individual values for methacholine $PC_{20}$ are plotted on a log scale in Figure 2. All subjects had duplicate $PC_{20}$ measurements within one doubling concentration. The geometric mean $PC_{20}$ was 1.78 mg/mL (95% CI, 0.95 to 3.4) with saline diluent and 1.84 mg/mL (95% CI, 0.97 to 3.5) with acetate diluent (p=0.76). There were small changes in $FEV_1$ following diluent inhalation. The percent fall in $FEV_1$ was $0.81 \pm 0.78$ (SE) % following saline inhalation and $1.3 \pm 0.62$% following acetate diluent inhalation; there was no significant difference between these two (p = 0.64). The mean pH of the various dilutions is shown in Table 29.

Table 29:

| pH of Methacholine Solutions | | | |
|---|---|---|---|
| Concentration | n | Conventional Formulation | Acetate based Formulation |
| diluent | 12 | 5.99 | 6.88 |
| 0.06 | 1 | 5.94 | 7.02 |
| 0.125 | 2 | 6.07 | 7.24 |
| 0.25 | 5 | 6.41 | 7.11 |
| 0.5 | 8 | 6.38 | 7.08 |
| 1 | 11 | 6.19 | 7.06 |
| 2 | 8 | 6.05 | 6.99 |
| 4 | 5 | 5.77 | 6.87 |

Table 29:   (continued)

| pH of Methacholine Solutions | | | |
|---|---|---|---|
| Concentration | n | Conventional Formulation | Acetate based Formulation |
| 8 | 2 | 6.17 | 6.8 |

CONCLUSION

These data demonstrate that the acetate diluent produces the same response in $FEV_1$ as the saline diluent. Methacholine in acetate diluent and methacholine in saline diluent produced identical responses. This data provide evidence that for the purpose of a methacholine challenge test, acetate diluent can be used interchangeably with saline diluent without altering patient response to methacholine.

All publications, patents and patent applications mentioned in this Specification are indicative of the level of skill of those skilled in the art to which this invention pertains.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

**Claims**

1.   An inhalable, stable formulation comprising methacholine chloride at a concentration of from 0.025 to 25 mg/ml dissolved in a buffer solution having a pH in the range of about 4 to about 5.

2.   The formulation according to claim 1, wherein the pH of the buffer solution is in the range of about 4.5 to about 5.0.

3.   The formulation according to claim 1, wherein the pH of the buffer solution is in the range of about 4.5 to about 4.7.

4.   The formulation according to any one of claims 1 - 3, wherein the buffer solution is an acetate or citrate buffer solution.

5.   The formulation according to claim 4, wherein the acetate buffer solution has an acetate concentration of about 4.5 to about 8.5 mM.

6.   The formulation according to claim 5, wherein the acetate concentration is about 4.8 mM.

7.   The formulation according to any one of claims 1-6, which has been filter sterilized by aseptic filtration.

8.   The formulation according to any one of claims 1-7, which additionally comprises a preservative.

9.   The formulation according to any one of claims 1 - 8, which additionally comprises sodium chloride.

10.  The formulation according to any one of claims 1-9, which is provided in a sealed container.

11.  The formulation according to claim 10, wherein the sealed container additionally comprises a stopper.

12.  The formulation according to any one of claims 1 - 11 for use in bronchoprovocation testing.

13.  A method of preparing the formulation according to claim 1 comprising the steps of:

    (a) dissolving methacholine chloride in the buffer solution; and
    (b) filter sterilising the solution formed in step (a) using aseptic filtration.

14.  The method according to claim 13, additionally comprising the step of preparing the buffer solution prior to step (a) by dissolving a buffer agent in water and, optionally, adjusting the pH to within the range of about 4 to about 5.

15.  The method according to claim 14, wherein the buffer agent is acetate, or a pharmaceutically acceptable salt thereof.

16. The method according to 15, wherein the buffer solution has an acetate concentration of about 4.5 to about 8.5 mM.

17. The method according to claim 16, wherein the acetate concentration is about 4.8 mM.

18. The method according to claim 14, wherein the buffer agent is citrate, or a pharmaceutically acceptable salt thereof.

19. A packaged pharmaceutical product comprising a formulation according to any one of claims 1 - 9 in a sealable container.

20. The packaged pharmaceutical product according to claim 19, wherein the container is a vial or an ampoule constructed of materials generally accepted for pharmaceutical preparations.

21. The packaged pharmaceutical product according to claim 20, wherein vial is sealed using a suitable closure system to ensure sterility.

22. A formulation as defined in any one of claims 1 - 11 for use in a diagnostic method.

23. A formulation according to claim 22 wherein said diagnostic method is a method for performing bronchoprovocation testing on a subject.

24. Use of methacholine chloride in the preparation of a formulation according to any one of claims 1 - 11 for performing bronchoprovocation testing on a subject.

25. A method for determining the amount of methacholine chloride present in a sample comprising the steps of:

   (a) injecting an aliquot of the sample into a high pressure liquid chromatography (HPLC) column;
   (b) monitoring the eluent from the HPLC column and estimating a peak area for methacholine from the sample;
   (c) injecting an aliquot of a standard solution of methacholine chloride into the HPLC column, which standard solution contains a known concentration of methacholine chloride;
   (d) monitoring the eluent from the HPLC column and estimating a peak area for methacholine from the standard solution; and, subsequently,
   (e) calculating from the peak areas obtained in steps (b) and (d) the amount of methacholine chloride in the sample,

   wherein, the HPLC column is a reverse phase column and the column is eluted using a mobile phase comprising tetramethylammonium chloride.

26. The method according to claim 25, wherein the mobile phase consists of a mobile phase consisting of a solution of 1-heptanesulfonic acid sodium, potassium phosphate and tetramethylammonium chloride in water containing 12% (v/v) methanol.

27. The method according to claim 26, wherein the potassium phosphate is potassium phosphate monobasic.

28. The method according to claim 25, wherein steps (a) to (d) are repeated, the estimated peak areas for the sample are used to calculate an average peak area for the sample, the estimated peak areas for the standard solution are used to calculate an average peak area for the standard solution and step (e) is performed using the average peak areas.

Figure 1

Figure 2